(19) Europäisches Patentamt
European Patent Office
Office européen des brevets

(11) Veröffentlichungsnummer: **0 302 329 B1**

(12) # EUROPÄISCHE PATENTSCHRIFT

(45) Veröffentlichungstag der Patentschrift: **29.01.92**

(51) Int. Cl.$^5$: **C07C 233/89**

(21) Anmeldenummer: **88111889.7**

(22) Anmeldetag: **23.07.88**

Die Akte enthält technische Angaben, die nach dem Eingang der Anmeldung eingereicht wurden und die nicht in dieser Patentschrift enthalten sind.

(54) **Verfahren zur Herstellung konzentrierter fliessfähiger wässriger Lösungen von Betainen.**

(30) Priorität: **07.08.87 DE 3726322**

(43) Veröffentlichungstag der Anmeldung:
**08.02.89 Patentblatt 89/06**

(45) Bekanntmachung des Hinweises auf die Patenterteilung:
**29.01.92 Patentblatt 92/05**

(84) Benannte Vertragsstaaten:
**AT BE CH DE ES FR GB IT LI NL SE**

(56) Entgegenhaltungen:
**EP-A- 0 020 907**
**DE-A- 2 750 731**

**CHEMICAL ABSTRACTS, Band 111, Nr. 8, 21. August 1989, Zusammenfassung Nr. 60017g, Columbus, Ohio, US; HU-A-45 488 (L. NAGY et al.) 28-07.1988**

(73) Patentinhaber: **Th. Goldschmidt AG**
**Goldschmidtstrasse 100 Postfach 101461**
**W-4300 Essen 1(DE)**

(72) Erfinder: **Bade, Volkbert, Dr.**
**Graffweg 38 b**
**W-4300 Essen 14(DE)**

**EP 0 302 329 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung fließfähiger wäßriger Lösungen von Betainen der allgemeinen Formel

$$R^1CONH(CH_2)_x\overset{\displaystyle R^2 \atop |}{\underset{| \atop \displaystyle R^3}{N^{(+)}}}-(CH_2)_yCOO^{(-)} \qquad I$$

| | |
|---|---|
| $R^1$ | Alkylrest einer Fettsäure mit 6 bis 18 Kohlenstoffatomen |
| $R^2$, $R^3$ | gleich oder ungleich, Alkylrest mit 1 bis 4 Kohlenstoffatomen |
| x | 2 oder 3 |
| y | 1, 2 oder 3 |

durch Quaternierung der Fettsäureamide

$R^1CONH(CH_2)_xNR^2R^3$ II

mit ω-Halogenalkylcarbonsäuren $X(CH_2)_yCOOY$ oder deren Salzen (X = Halogenrest, Y = Wasserstoff-, Alkali- oder Ammoniumion) in wäßriger Lösung bei erhöhten Temperaturen.

Die Erfindung betrifft insbesondere die Herstellung von fließfähigen Betainlösungen mit einem Feststoffgehalt von 43 bis etwa 65 Gew.-% und einem Betaingehalt von 37 bis etwa 55 Gew.-%.

Die Erfindung betrifft besonders bevorzugt die Herstellung fließfähiger Betainlösungen der vorgenannten Konzentration, welche homogen sind und eine hohe Kältestabilität aufweisen.

Betaine der Formel I haben in den vergangenen Jahren zunehmende Bedeutung zur Herstellung von Körperreinigungsmitteln erlangt. Sie verbinden ausgezeichnete Reinigungseigenschaften mit guter Hautverträglichkeit. Die Betaine bilden in wäßriger Lösung einen stabilen dichten Schaum aus, der auch in Gegenwart von Seife nicht zusammenfällt.

Die Herstellung dieser Betaine ist in vielen Patentschriften beschrieben, von denen die US-PS 3 225 074 stellvertretend genannt wird. Im allgemeinen setzt man dabei das entsprechende tertiäre Fettsäureamidamin der allgemeinen Formel II mit dem Alkalisalz einer ω-Halogencarbonsäure, in der Regel dem Natriumsalz der Chloressigsäure, um. Die Reaktion läuft im wäßrigen Medium ab. Das bei der Reaktion entstehende Alkalichlorid wird im allgemeinen nicht aus der Lösung entfernt.

Die Betaine des Standes der Technik werden meist in Form ihrer 30 gew.-%igen wäßrigen Lösungen in den Handel gebracht. Es hat nicht an Versuchen gefehlt, höher konzentrierte Lösungen der Betaine herzustellen, um den Transport und die Lagerung der Betainlösungen zu verbilligen. Entzieht man jedoch den nach dem Stand der Technik erhältlichen Betainlösungen Wasser, steigt die Viskosität der Lösungen schnell an. Lösungen von Betainen auf Basis gehärteter Kokosfettsäure werden bei einem Gehalt von etwa 35 bis 37 Gew.-% Betain pastös und verfestigen sich bei weiterer Entwässerung zunehmend. Die Konzentration, von der ab Betainlösungen nicht mehr fließfähig sind, wird durch die Kohlenstoffanzahl der Fettsäure $R^1COOH$ beeinflußt. Je größer die Kettenlänge der Fettsäure oder des zur Herstellung des Betains verwendeten Fettsäuregemisches ist, um so schneller steigt die Viskosität der Betainlösungen bei steigender Konzentration an. Ungesättigte Fettsäuren gleicher Kohlenstoffanzahl ergeben Betainlösungen niedriger Viskosität.

Es ist dem Fachmann zwar bekannt, daß mit steigender Konzentration einer wäßrigen Tensidlösung auch die Viskosität ansteigt. Häufig zeigt sich jedoch bei Überschreiten einer Konzentration von etwa 60 bis 70 Gew.-%, daß die Viskosität bei weiterer Konzentrationserhöhung auf ein Minimum abfällt, um dann erneut stark anzusteigen. Zur Erklärung dieser Viskositätsanomalie nimmt man an, daß sich in der Lösung eine G-Phase mit lamellarer Struktur ausbildet. (Soap, Perfumery, Cosmetics 1982, 507 bis 509). Ein solches Verhalten konnte aber bei Betainen nicht beobachtet werden. Auch bei weiterem Wasserentzug verflüssigen sich die verfestigten Produkte nicht mehr.

In der DE-OS ....... (P 36 13 944.0-42) ist ein Verfahren zur Herstellung einer fließ- und pumpfähigen, mindestens 70 Gew.-% Betain der Formel I enthaltenden Lösung beschrieben, bei dem man

a) als Salz der Halogencarbonsäure das Ammoniumsalz einsetzt,

b) die Quaternierung in einem organischen polaren Lösungsmittel, welches höchstens 20 Gew.-%

Wasser enthalten darf, durchführt,

c) nach der Quaternierung das gegebenenfalls enthaltene Wasser azeotrop abdestilliert und das ausgefallene Ammoniumhalogenid abtrennt, danach

d) das Lösungsmittel ganz oder teilweise abdestilliert und

e) vor, gleichzeitig oder nach der Destillation die gewünschte Konzentration des Betains in dem anwendungstechnisch gewünschten Lösungsmittel oder Lösungsmittelgemisch einstellt.

Bei diesem Verfahren muß somit die Quaternierung in einem organischen polaren Lösungsmittel durchgeführt werden. Als Verfahrensprodukt liegt die konzentrierte Lösung des Betains in einem anwendungstechnisch gewünschten Lösungsmittel oder Lösungsmittelgemisch vor.

Es besteht jedoch nach wie vor der Bedarf, möglichst konzentrierte wäßrige Lösungen von Betainen der Formel I herzustellen, welche trotz ihrer vergleichsweise hohen Konzentration fließ- und pumpfähig und vorzugsweise frei von Lösungsmitteln außer Wasser sind. Die niedrige Viskosität der wäßrigen Lösungen ist insbesondere erforderlich, um die wäßrigen Betainlösungen bei der Weiterverarbeitung fördern und dosieren zu können. Darüber hinaus besteht ein erhebliches wirtschaftliches Interesse, die Verpackungs-, Transport- und Lagerkosten sowie den Handhabungsaufwand zu erniedrigen. Dabei ist es von besonderer Wichtigkeit, daß die konzentrierten wäßrigen Lösungen beim Verdünnen ohne Gelbildung in Wasser verteilt werden können.

Überraschenderweise wurde nun gefunden, daß man fließfähige Lösungen von Betainen der allgemeinen Formel I durch Quaternierung der Fettsäureamide $R^1CONH(CH_2)_xNR^2R^3$ mit $\omega$-Halogenalkylcarbonsäuren $X(CH_2)_yCOOY$ oder deren Salzen (X = Halogenrest, Y = Wasserstoff-, Alkali- oder Ammoniumion) in wäßriger Lösung bei erhöhten Temperaturen dadurch herstellen kann, daß man die nach der Umsetzung erhaltene, vorzugsweise noch heiße Lösung, falls erforderlich, durch Abdampfen von Wasser auf die gewünschte Konzentration einstellt und vor oder nach der Einstellung der gewünschten Konzentration der Lösung Mineralsäure in solchen Mengen zusetzt, daß der pH-Wert der Lösung 1 bis 4,5 beträgt.

Als Mineralsäure können vorzugsweise Salz-, Schwefel- oder Phosphorsäure verwendet werden.

Die Zugabe der Mineralsäure erfolgt nach der Quaternierungsreaktion. Will man die zunächst erhaltene Lösung durch Einengen, gegebenenfalls bei vermindertem Druck, konzentrieren, setzt man die Mineralsäure vorzugsweise bereits vor dem Einengen zu, um die Bildung hochviskoser Lösungen zu vermeiden. Es ist aber durchaus möglich, die Mineralsäure der aufkonzentrierten, gelartigen Lösung zuzusetzen, die sich beim Einrühren der Mineralsäure verflüssigt.

Die Quaternierungsreaktion kann in an sich bekannter Weise durchgeführt werden. Man setzt dabei dem Fettsäureamidamin der Formel II in wäßrigem Medium das Alkalisalz, vorzugsweise das Natriumsalz der Monochloressigsäure, zu und läßt die Quaternierungsreaktion im Temperaturbereich von etwa 80 °C bis zur Rückflußtemperatur des Reaktionsgemisches ablaufen. Es ist auch möglich, zunächst die Monochloressigsäure in dem Wasser zu lösen und in die Lösung eine Schmelze des Fettsäureamidamins der Formel II einzurühren. Es erfolgt eine exotherme Reaktion, nach deren Abklingen die zur Neutralisation erforderliche Menge Alkalilauge langsam zugegeben wird. Das letztere Verfahren (Säureverfahren) ermöglicht die Herstellung etwas höher konzentrierter Lösungen bei gleicher Viskosität.

Verwendet man ein aus gehärtetem Kokosfett erhaltenes Fettsäuregemisch zur Hersellung des Fettsäureamidamins der Formel I, kann man nach dem Stand der Technik Betainlösungen erhalten, die bis zu der angegebenen Konzentration (in Gew.-%) noch fließfähig sind, darüber aber vergelen bzw. sich verfestigen und dann nicht mehr handhabbar sind:

Stand der Technik (Vergleich)

| Quaternierung mit | Festkörpergehalt | Betaingehalt |
|---|---|---|
| $ClCH_2COONa$ | 42 | 36 |
| $ClCH_2COOH/NaOH$ (Säureverfahren) | 44 | 38 |
| $ClCH_2COOH/KOH$ (Säureverfahren) | 47 | 39 |

Setzt man aber erfindungsgemäß diesen Lösungen Mineralsäure in solchen Mengen zu, daß der pH-Wert der Lösungen innerhalb des Bereiches von 1 bis 4,5 liegt, lassen sich folgende Grenzkonzentrationen bei Verwendung des gleichen Fettsäuregemisches erzielen:

Erfindungsgemäßes Verfahren

| Quaternierung mit | Mineralsäure | ph-Wert | Festkörpergehalt | Betaingehalt |
|---|---|---|---|---|
| $ClCH_2COONa$ | HCl | 4,5 | 51 - 53 | 41 - 43 |
| $ClCH_2COOH/KOH$ | HCl | 4,4 | 53 - 54 | 43 - 44 |
| $ClCH_2COOH/KOH$ | HCl | 3,3 | 53 - 54 | 43 - 44 |
| $ClCH_2COOH/KOH$ | HCl | 1,4 | 61 | 46 - 47 |
| $ClCH_2COOH/KOH$ | $H_3PO_4$ | 4,0 | 54 - 55 | 43 - 44 |
| $ClCH_2COOH/KOH$ | $H_2SO_4$ | 2,5 - 3,0 | 54 | 43 - 44 |

Die Tabelle zeigt, daß es bei dieser Ausführungsform des erfindungsgemäßen Verfahrens möglich ist, noch fließfähige Lösungen herzustellen, deren Betaingehalt um mindestens 10 % höher als bei entsprechenden Lösungen des Standes der Technik ist.

In der folgenden Tabelle wird der Einfluß der Zusammensetzung des Fettsäuregemisches, das zur Herstellung des Fettsäureamidamins der Formel I verwendet worden ist, auf die Grenzkonzentration noch fließfähiger Betainlösungen eines pH-Wertes von etwa 3 gezeigt:

| Fettsäure oder Fettsäuregemisch | maximaler Festkörpergehalt in Gew.-% |
|---|---|
| $C_8$-Fettsäure | 60 bis 61 |
| $C_{10}$-Fettsäure | 53 |
| $C_{12}$-Fettsäure | 44 |
| $C_{14}$-Fettsäure | 38 |
| Kokosfettsäure, gestrippt | 43 |
| Kokosfettsäure, gehärtet | 54 |
| Kokosfettsäure, gehärtet + Palmkernfettsäure, gehärtet, 1: 1 gemischt | 54 |
| wie vorstehend, aber 3 : 1 gemischt | 54 |

Aus der Tabelle ist auch ersichtlich, daß es bei Verwendung von Fettsäuregemischen möglich ist, Lösungen höherer Konzentration herzustellen, als dies bei Verwendung einer reinen Fettsäure, deren Kohlenstoffzahl der mittleren Kohlenstoffzahl des Fettsäuregemisches entspricht, gelingt.

Eine bevorzugte Ausführungsform des erfindungsgemäßen Verfahrens ist dadurch gekennzeichnet, daß man dem Reaktionsgemisch vor oder während der Quaternierung oder der erhaltenen Betainlösung vor deren Einstellung auf einen pH-Wert von 1 bis 4,5 1 bis 5 Gew.-%, bezogen auf Lösung, wasserlösliche nichtionogene Tenside zusetzt und gegebenenfalls die so erhaltene saure Lösung der Betaine durch Zugabe von Lauge auf einen pH-Wert von $\geqq$ 5 bis 9 einstellt.

Durch den Zusatz der nichtionogenen Tenside wird die erfindungsgemäß erhaltene Betainlösung bezüglich ihrer Verarbeitungseigenschaften mehrfach verbessert: In der Lösung gelegentlich vorhandene Trübungen und Schlieren werden solubilisiert, so daß die Lösungen optisch blank werden. Die Lösungen scheiden auch bei längerem Stehen keine Trübungen mehr aus. Sie weisen eine hervorragende Kältestabilität auf. Gleichzeitig wird die Viskosität der Lösungen noch weiter erniedrigt.

Als wasserlösliche nichtionogene Tenside sind die Polyoxyalkylenderivate von Fettalkoholen, Fettsäuren oder partiellen Fettsäureestern mehrwertiger Alkohole bevorzugt. Beispiele derartiger, bevorzugter Verbindungen sind die Polyoxyethylenether von Fettalkoholen mit 8 bis 18 Kohlenstoffatomen, wobei der Fettalkohol gesättigt oder ungesättigt, substituiert oder unsubstituiert sein kann. Beispiele solcher Fettalkohole sind der Lauryl-, Stearyl- und Oleylalkohol. Als nichtionogene Tenside sind ferner die Polyoxyethylenester von Fettsäuren geeignet. Beispiele geeigneter Fettsäuren sind Laurin-, Stearin-, Öl- und Rizinusfettsäure sowie aus natürlichen Fetten gewonnene Fettsäuregemische. Weiter geeignet sind die ethoxylierten Fettsäure-mono- und -diglyceride sowie die entsprechenden ethoxylierten Sorbitfettsäureester. Der HLB-Wert dieser nichtionogenen Tenside soll insbesondere in einem Bereich von 14 bis 20 liegen.

Als nichtionogene Tenside sind auch die Alkylpolyglukoside und deren Alkoxylierungsprodukte geeignet. Besonders geeignet sind Alkylglukoside, deren Alkylreste 8 bis 12 C-Atome aufweisen.

Die nichtionogenen Tenside können bereits dem Reaktionsgemisch zur Herstellung der Betaine zugesetzt werden. In diesem Falle sind jedoch nur solche Tenside brauchbar, die unter den Reaktionsbedingungen nicht gespalten (verseift) werden, wie etwa die Polyoxyethylenether der Fettalkohole. Vorzugsweise gibt man die Tenside in die erhaltene Betainlösung. Jedoch muß die Zugabe vor dem Ansäuern der Betainlösung entsprechend dem erfindungsgemäßen Verfahren erfolgen.

Im allgemeinen genügt ein Zusatz von 1 bis 5 Gew.-%, bezogen auf Lösung, um den gewünschten Effekt zu erzielen. Der Zusatz der nichtionogenen Tenside stört bei der weiteren Verwendung der Betainlösungen nicht, da die Betainlösungen bei ihrer Verwendung zur Herstellung von Shampoos oder Duschgelen im allgemeinen mit nichtionogenen Tensiden compoundiert werden. Der Zusatz von 1 bis 5 Gew.-% nichtionogener Tenside ist deshalb nur als Mindestmenge zu verstehen, um die gewünschte Kältestabilität zu gewährleisten und die niedrige Viskosität der Lösungen sicherzustellen. Es ist selbstverständlich möglich, den Betainlösungen auch größere Mengen an nichtionogenen Tensiden zuzusetzen.

Es hat sich überraschend gezeigt, daß eine erfindungsgemäß hergestellte saure Betainlösung, welche nichtionogene Tenside enthält, ohne die Gefahr einer Vergelung auf einen pH-Wert von ≧ 5 bis 7 eingestellt werden kann. Dies ist insbesondere im Hinblick auf verminderte Korrosivität der Lösungen von Bedeutung. Neutralisiert man eine erfindungsgemäß hergestellte Betainlösung, die frei von nichtionogenen Tensiden ist, vergelt diese, wie folgende Tabelle zeigt:

Die Lösung enthält 53 bis 54 Gew.-% Feststoff, davon 43 bis 44 Gew.-% Betain auf Basis von gehärteter Kokosfettsäure und ist mit HCl auf einen pH-Wert von 3,3 eingestellt. Es wird eine Lösung ohne nichtionogenes Tensid mit einer Lösung mit 4,1 Gew.-% ethoxyliertem Sorbitanmonolaurinsäureester verglichen:

| | ohne Tensid | mit Tensid |
|---|---|---|
| Viskosität<br>Aussehen<br>Kältestabilität<br>nach Neutralisation | 1 000 mPas bei 25 °C<br>leicht trüb, Schlieren<br>< 16 °C gelartig<br>Vergelung | 600 mPas bei 25 °C<br>klar<br>bis 0 °C klar, flüssig<br>bis pH 9 flüssig |

Das erfindungsgemäße Verfahren gestattet es somit, flüssige pump- und dosierfähige Betainlösungen, z. B. auf Basis von Kokosfettsäure, mit einem Betaingehalt von mindestens 37 bis etwa 50 Gew.-% herzustellen.

Die erfindungsgemäß hergestellten Lösungen lassen sich ohne weiteres verdünnen und bilden beim Verdünnen keine Gelstrukturen. Die Betainlösungen können in üblicher Weise konfektioniert werden. Dabei soll unter dem Begriff Konfektionierung die Einstellung der gewünschten Konzentration, gegebenenfalls Zusatz von Farbstoffen, Parfümierungsmitteln, anderen hautpflegenden Substanzen und/oder Verdickungsmitteln verstanden werden.

In den folgenden Beispielen wird das erfindungsgemäße Verfahren noch weiter erläutert.

Beispiel 1 (nicht erfindungsgemäßer Vergleich)

In einen 1-l-Vierhalskolben mit Rührer, Thermometer und Rückflußkühler werden 300 g Fettsäureamidamin der Formel II ($R^1$ = Alkylrest eines aus gehärtetem Kokosfett erhaltenen Fettsäuregemisches $R^1COOH$, $R^2$, $R^3$ = Methylreste, x = 3, y = 2), 128 g Natriumchloracetat und 590 g Wasser eingewogen. Das Reaktionsgemisch wird auf 95 bis 98 °C erwärmt. Nach einer Reaktionszeit von 10 bis 12 Stunden beträgt der Gehalt an nicht umgesetztem Fettsäureamidamin < 2 Gew.-%. Es werden 1 018 g eines flüssigen, klaren, homogenen Produktes mit einem pH-Wert von 5 erhalten.

Feststoffgehalt: 42 Gew.-%

Betaingehalt: 36 Gew.-%

Die erhaltene Lösung wird durch Abdampfen eingeengt. Bei Erreichen eines Feststoffgehaltes von 43 bis 44 Gew.-% ist ein festes Gel entstanden.

Beispiel 2 (nicht erfindungsgemäßer Vergleich)

In einer Apparatur gemäß Beispiel 1 werden 103 g Monochloressigsäure bei 60 bis 70 °C in 458 g Wasser gelöst. 300 g des in Beispiel 1 genannten Fettsäureamidamins werden bei 30 bis 40 °C aufgeschmolzen und zu der Lösung der Monochloressigsäure gegeben. Es erfolgt eine exotherme Reaktion. Die erhaltene Lösung wird auf 75 bis 80 °C abgekühlt. Bei dieser Temperatur läßt man während einer Stunde 44 g NaOH, gelöst in 44 g Wasser, zulaufen. Man erhöht die Temperatur auf 95 bis 98 °C und läßt 4 bis 6 Stunden nachreagieren, bis der Gehalt an Fettsäureamidamin auf < 2 Gew.-% gesunken ist. Es werden 949 g eines flüssigen, klaren, homogenen Produktes mit einem pH-Wert von 5 erhalten.

Feststoffgehalt: 44 Gew.-%

Betaingehalt: 38 Gew.-%

Die erhaltene Lösung wird durch Abdampfen eingeengt. Bei Erreichen eines Feststoffgehaltes von 46 Gew.-% ist ein festes Gel entstanden.

Beispiel 3 (nicht erfindungsgemäßer Vergleich)

In einer Apparatur gemäß Beispiel 1 werden 103 g Monochloressigsäure bei 60 bis 70 °C in 420 g Wasser gelöst. 300 g des in Beispiel 1 genannten Fettsäureamidamins werden bei 30 bis 40 °C aufgeschmolzen und zu der Lösung der Monochloressigsäure gegeben. Es erfolgt eine exotherme Reaktion. Die erhaltene Lösung wird auf 75 bis 80 °C abgekühlt. Bei dieser Temperatur läßt man während einer Stunde 73 g 85 gew.-%ige KOH, gelöst in 50 g Wasser, zulaufen. Man erhöht die Temperatur auf 95 bis 98 °C und läßt 4 bis 6 Stunden nachreagieren, bis der Gehalt an Fettsäureamidamin auf < 2 Gew.-% gesunken ist. Es werden 949 g eines flüssigen, klaren, homogenen Produktes mit einem pH-Wert von 5 erhalten.
Feststoffgehalt: 47 Gew.-%
Betaingehalt: 39 Gew.-%

Die erhaltene Lösung wird durch Abdampfen eingeengt. Bei Erreichen eines Feststoffgehaltes von 48 Gew.-% ist ein festes Gel entstanden.

Beispiel 4 (erfindungsgemäß)

Es wird das Verfahren des Beispiels 3 wiederholt. Nach Reaktionsende wird über die erhaltene Lösung Stickstoff geleitet und der Rückflußkühler entfernt. Es werden 15 g 36%iger wäßriger HCl zugesetzt und anschließend soviel Wasser abgedampft, bis ein Feststoffgehalt von 53 bis 54 Gew.-% ermittelt wird. Es werden 827 g einer flüssigen, schwach trüben Lösung erhalten.
Feststoffgehalt: 54 Gew.-%
Betaingehalt: 44 Gew.-%
pH-Wert: 4,4

Beispiel 5 (erfindungsgemäß)

Es wird das Verfahren des Beispiels 4 wiederholt. Es werden jedoch 30 g 36%iger wäßriger HCl zugesetzt. Es wird bis zu einem Feststoffgehalt von 54 Gew.-% eingeengt. Es werden 842 g einer flüssigen, trüben Lösung erhalten.
Feststoffgehalt: 54 Gew.-%
Betaingehalt: 44 Gew.-%
pH-Wert: 3,3

Beispiel 6 (erfindungsgemäß)

Es wird das Verfahren des Beispiels 4 wiederholt. Es werden jedoch 100 g 36%iger wäßriger HCl zugesetzt. Es wird bis zu einem Feststoffgehalt von 61 Gew.-% eingeengt. Es werden 790 g einer dickflüssigen, aber gießfähigen, trüben Lösung erhalten.
Feststoffgehalt: 61 Gew.-%
Betaingehalt: 46 bis 47 Gew.-%
pH-Wert: 1,4

Beispiel 7 (erfindungsgemäß)

Es wird das Verfahren des Beispiels 4 wiederholt. Es werden jedoch 10 g 85%iger wäßriger Phosphorsäure zugesetzt. Es wird bis zu einem Feststoffgehalt von 54 Gew.-% eingeengt. Es werden 840 g einer flüssigen, trüben Lösung erhalten.
Feststoffgehalt: 54 Gew.-%
Betaingehalt: 44 Gew.-%
pH-Wert: 4

Beispiel 8 (erfindungsgemäß)

Es wird das Verfahren des Beispiels 4 wiederholt. Es werden jedoch 10 g 98%iger Schwefelsäure

zugesetzt. Es wird bis zu einem Feststoffgehalt von 54 Gew.-% eingeengt. Es werden 840 g einer flüssigen, trüben Lösung erhalten.
Feststoffgehalt: 54 Gew.-%
Betaingehalt: 43 bis 44 Gew.-%
pH-Wert: 2,5 bis 3

Beispiel 9 (erfindungsgemäß)

In einer Apparatur gemäß Beispiel 1 werden 103 g Monochloressigsäure bei 60 bis 70 °C in 400 g Wasser gelöst. 300 g des in Beispiel 1 verwendeten Fettsäureamidamins werden bei 30 bis 40 °C aufgeschmolzen und zu der Lösung gegeben. Es erfolgt eine exotherme Reaktion. Die erhaltene Lösung wird auf 75 bis 80 °C abgekühlt. Bei dieser Temperatur läßt man während einer Stunde eine Lösung von 73 g KOH (85%ig) in 50 g Wasser zulaufen.

Zu diesem Reaktionsgemisch gibt man 100 g aufgeschmolzenes Fettsäureamidamin und 34 g Monochloressigsäure. Man läßt bei einer Temperatur von 75 bis 80 °C eine Lösung von 24 g KOH (85%ig) in 17 g Wasser zulaufen. Die Temperatur der Lösung wird dann auf 95 bis 98 °C erhöht. Nach 4 bis 6 Stunden ist der Gehalt an freiem Fettsäureamidamin auf < 2 Gew.-% gesunken. Man setzt 40 g 36%iger wäßriger HCl zu und läßt abkühlen. Es werden 1 140 g eines flüssigen, klaren, homogenen Produktes mit einem pH-Wert von 3,3 erhalten.
Feststoffgehalt: 54 Gew.-%
Betaingehalt: 44 Gew.-%
pH-Wert: 3,3

Beispiel 10 (erfindungsgemäß)

Es wird das Verfahren des Beispiels 5 wiederholt, jedoch dem Umsetzungsprodukt vor dem Ansäuern 50 g einer 70 gew.-%igen wäßrigen Polyoxyethylensorbitanmonolaurat-Lösung zugegeben. Anschließend werden wie in Beispiel 5 30 g 36 gew.-%iger HCl zugesetzt. Die Lösung wird unter leichtem Erwärmen auf 30 bis 35 °C so lange gerührt, bis eine klare Lösung entstanden ist. Die erhaltene Lösung wird dann mit solchen Mengen 47 gew.-%iger wäßriger KOH-Lösung versetzt, bis ein pH-Wert von 5 resultiert. Ein Vergleich mit dem Produkt von Beispiel 5 ergibt:

| | ohne Tensid | mit Tensid |
|---|---|---|
| Viskosität | 1 000 mPas bei 25 °C | 600 mPas bei 25 °C |
| Aussehen | leicht trüb, Schlieren | klar |
| Kältestabilität | < 16 °C gelartig | bis 0 °C klar, flüssig |
| nach Neutralisation | Vergelung | bis pH 9 flüssig |

Beispiel 11

Es wird das Verfahren des Beispiels 5 wiederholt. Es werden jedoch zur Herstellung der Betaine Fettsäureamidamine verwendet, welche von verschiedenen Fettsäuren oder Fettsäuregemischen abgeleitet sind. Es werden die Konzentrationen ermittelt, bei denen die erhaltenen Lösungen, welche einen pH-Wert von etwa 3 aufweisen, noch flüssig sind:

| Fettsäure oder Fettsäuregemisch | maximaler Festkörpergehalt in Gew.-% |
|---|---|
| $C_8$-Fettsäure | 60 bis 61 |
| $C_{10}$-Fettsäure | 53 |
| $C_{12}$-Fettsäure | 44 |
| $C_{14}$-Fettsäure | 38 |
| Kokosfettsäure, gestrippt | 43 |
| Kokosfettsäure, gehärtet | 54 |
| Kokosfettsäure, gehärtet + Palmkernfettsäure, gehärtet, 1: 1 gemischt | 54 |
| wie vorstehend, aber 3 : 1 gemischt | 54 |

Beispiel 12

In einer Apparatur gemäß Beispiel 1 werden 103 g Monochloressigsäure bei 60 bis 70 °C in 400 g Wasser gelöst. 300 g des in Beispiel 1 verwendeten Fettsäureamidamins werden bei 30 bis 40 °C aufgeschmolzen und zu der Lösung gegeben. Es erfolgt eine exotherme Reaktion. Die erhaltene Lösung wird auf 75 bis 80 °C abgekühlt. Bei dieser Temperatur läßt man während einer Stunde eine Lösung von 73 g KOH (85%ig) in 50 g Wasser zulaufen.

Zu diesem Reaktionsgemisch gibt man 100 g aufgeschmolzenes Fettsäureamidamin, 34 g Monochloressigsäure und 57 g Polyoxyethylenlaurylether. Man läßt bei einer Temperatur von 75 bis 80 °C eine Lösung von 24 g KOH (85%ig) in 17 g Wasser zulaufen. Die Temperatur der Lösung wird dann auf 95 bis 98 °C erhöht. Nach 4 bis 6 Stunden ist der Gehalt an freiem Fettsäureamidamin auf < 2 Gew.-% gesunken. Man setzt 40 g 36%iger wäßriger HCl zu und läßt abkühlen. Es werden 1 200 g eines flüssigen, klaren, homogenen Produktes mit einem pH-Wert von 3,3 erhalten.
Feststoffgehalt: 55 Gew.-%
Betaingehalt: 44 Gew.-%
pH-Wert: 4

Die erhaltene Lösung wird mit wäßriger KOH-Lösung neutralisiert. Die Lösung bleibt flüssig und klar. Sie kann auf 5 °C ohne Verlust ihrer Fließfähigkeit und ohne Eintrübung abgekühlt werden.

**Patentansprüche**

**1.** Verfahren zur Herstellung fließfähiger wäßriger Lösungen von Betainen der allgemeinen Formel

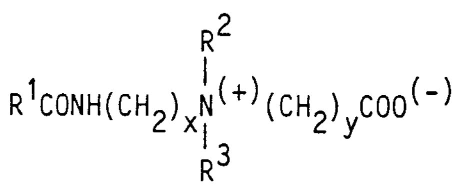

| | |
|---|---|
| $R^1$ | Alkylrest einer Fettsäure mit 6 bis 18 Kohlenstoffatomen |
| $R^2$, $R^3$ | gleich oder ungleich, Alkylrest mit 1 bis 4 Kohlenstoffatomen |
| x | 2 oder 3 |
| y | 1, 2 oder 3 |

durch Quaternierung der Fettsäureamide

$R^1CONH(CH_2)_xNR^2R^3$

mit ω-Halogenalkylcarbonsäuren $X(CH_2)_yCOOY$ oder deren Salzen (X = Halogenrest, Y = Wasserstoff-, Alkali- oder Ammoniumion) in wäßriger Lösung bei erhöhten Temperaturen, dadurch gekennzeichnet, daß man die nach der Umsetzung erhaltene, vorzugsweise noch heiße Lösung falls erforderlich durch Abdampfen von Wasser auf die gewünschte Konzentration einstellt und vor oder nach der Einstellung der gewünschten Konzentration der Lösung Mineralsäure in solchen Mengen zusetzt, daß der pH-Wert der Lösung 1 bis 4,5 beträgt.

**2.** Weiterausbildung des Verfahrens nach Anspruch 1, dadurch gekennzeichnet, daß man dem Reaktionsgemisch vor oder während der Quaternierung oder der erhaltenen Betainlösung vor deren Einstellung auf einen pH-Wert von 1 bis 4,5 1 bis 5 Gew.-%, bezogen auf Lösung, wasserlösliche nichtionogene Tenside zusetzt und gegebenenfalls die so erhaltene saure Lösung der Betaine durch Zugabe von Lauge auf einen pH-Wert von ≧ 5 bis 9 einstellt.

**3.** Verfahren nach Anspruch 2, dadurch gekennzeichnet, daß man als wasserlösliche nichtionogene Tenside Polyoxyethylenderivate von Fettalkoholen, Fettsäuren oder partiellen Fettsäureestern mehrwertiger Alkohole mit einem HLB-Wert von 14 bis 20 zusetzt.

**Claims**

**1.** Process for the preparation of free-flowing aqueous solutions of betaines of the general formula

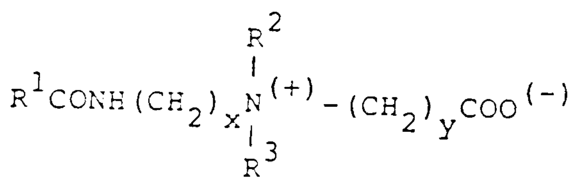

in which

| | |
|---|---|
| $R^1$ | is an alkyl radical of a fatty acid having 6 to 18 carbon atoms, |
| $R^2$ and $R^3$ | are identical or non-identical alkyl radicals having 1 to 4 carbon atoms, |
| x | is 2 or 3 and |
| y | is 1, 2 or 3 |

by quaternisation of the fatty acid amides

$R^1CONH(CH_2)_xNR^2R^3$

with ω-haloalkylcarboxylic acids $X(CH_2)_yCOOY$ or their salts (X = halogen radical, Y = hydrogen, alkali metal or ammonium ion) in aqueous solution at elevated temperatures, characterised in that the solution which is obtained after the reaction and is preferably still hot is adjusted to the desired concentration, if necessary by evaporation of water, and mineral acid is added before or after establishing the desired concentration in amounts such that the pH of the solution is 1 to 4.5.

**2.** Further improvements of the process according to Claim 1, characterised in that 1 to 5% by weight, relative to the solution, of water-soluble non-ionic surfactants are added to the reaction mixture before or during the quaternisation or to the resultant betaine solution before its adjustment to a pH of 1 to 4.5 and, if desired, the acidic solution of the betaines thus obtained is adjusted to a pH of ≧ 5 to 9 by addition of alkali metal hydroxide solution.

**3.** Process according to Claim 2, characterised in that the water-soluble non-ionic surfactants added are polyoxyethylene derivatives of fatty alcohols, fatty acids or partial fatty acid esters of polyhydric alcohols having an HLB of 14 to 20.

**Revendications**

**1.** Procédé de préparation de solutions de bétaïnes aqueuses et fluides, répondant à la formule générale

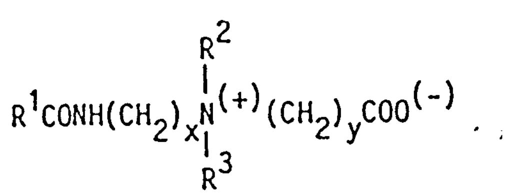

dans laquelle

| | |
|---|---|
| $R^1$ | représente un radical alcoyle d'un acide gras comportant 6 à 18 atomes de carbone, |
| $R^2$ et $R^3$ | qui sont identiques ou différents, représentent un radical alcoyle comportant 1 à 4 atomes de carbone, |
| x | vaut 2 ou 3, |
| y | vaut 1, 2 ou 3, |

par quaternisation des amides d'acides gras

$R^1CONH(CH_2)_xNR^2R^3$

avec des acides ω-halogénalcoylcarboxyliques $X(CH_2)_yCOOY$ ou leurs sels (X = radical halogène, Y = ion hydrogène, alcalin ou ammonium) en solution aqueuse, à des températures élevées, caractérisé en ce qu on règle à la concentration souhaitée la solution obtenue après la réaction, de préférence encore chaude, si nécessaire par évaporation d'eau, et avant ou après le réglage de la solution à la concentration souhaitée, on ajoute un acide minéral en quantités telles que le pH de la solution soit compris entre 1 et 4,5.

**2.** Perfectionnement du procédé selon la revendication 1, caractérisé en ce qu'on ajoute au mélange réactionnel, avant ou pendant la quaternisation, ou à la solution de bétaïnes obtenue, avant de la régler à pH 1 à 4,5, 1 à 5 % en poids, par rapport au poids de la solution, d'agents tensio-actifs non ionogènes solubles dans l'eau et, éventuellement, on règle la solution acide des bétaïnes ainsi obtenue a pH $\geqq$ 5 à 9, par addition d'une lessive.

**3.** Procédé selon la revendication 2, caractérisé en ce qu'on ajoute, comme agents tensio-actifs non ionogènes, des dérivés polyoxyéthyléniques d'alcools gras, d'acides gras ou d'esters partiels d'acides gras de polyols ayant une valeur HLB de 14 à 20.